# EUROPEAN PATENT SPECIFICATION

(11) **EP 4 065 205 B1**
(45) Date of publication and mention of the grant of the patent: **07.08.2024**
(21) Application number: 20815911.1
(22) Date of filing: 24.11.2020
(51) Int. Cl.: A61M 25/00, A61M 25/06, A61M 25/01

(54) **GUIDING DEVICE FOR A VASCULAR CATHETER**
LEITVORRICHTUNG FÜR EINEN GEFÄSSKATHETER
DISPOSITIF DE GUIDAGE POUR UN CATHÉTER VASCULAIRE

(30) Priority: 26.11.2019 GB 201917184
(43) Date of publication of application: 05.10.2022
(73) Proprietor: The University Of Sheffield, Sheffield, South Yorkshire S10 2TN (GB)
(72) Inventor: GUNN, Julian Philip George, Sheffield South Yorkshire S10 2RX (GB); MORRIS, Paul David, Sheffield South Yorkshire S10 2RX (GB)
(74) Representative: HGF
(86) International application number: PCT/GB2020/052985
(87) International publication number: WO 2021/105658

(56) References cited:
- US-A- 5 976 153
- US-A1- 2006 074 400
- US-A1- 2009 264 863
- US-A1- 2016 220 795
- US-A1- 2019 247 619
- US-A1- 2019 255 297
- US-A1- 2019 351 182

## Description

This invention relates to a guiding device for a vascular catheter, and in particular, to a guiding device that may facilitate improved passage of a catheter through a blood vessel.

### BACKGROUND

Arterial disease is currently the leading cause of death in the UK and the world. It arises due to a narrowing resulting from a build-up of fatty deposits (atherosclerosis). The coronary arteries run along the surface of the heart and supply blood to the heart muscle. The narrowing(s) associated with coronary heart disease, for example, limit blood flow and this can starve the heart muscle of its vital oxygen supply. To gain access to the coronary and other arteries, medical practitioners need to insert a catheter into the vascular system. For access to the coronary arteries, for the commonest of vascular interventions, the catheter was traditionally inserted via the femoral artery. More recently, it has become more common for the radial artery to be used. The most common procedure to treat coronary heart disease is called coronary angioplasty or percutaneous coronary intervention (PCI) (commonly known as 'stenting').

In such a procedure, the narrowing in the coronary artery is identified by injecting contrast dye under X-ray guidance. A balloon is inflated to relieve the narrowing and a stent (a metal mesh tube) inserted. The stent acts to keep the artery open and blood flow is restored. In the UK, around 100,000 PCI procedures are performed per annum.

A catheter is used to deliver the contrast dye and angioplasty equipment including wires, balloons and stents. Because of the technical demands placed on a vascular catheter, it is often made from a relatively stiff material and is formed into a cylindrical tube. Advancing the vascular catheter through the artery in the arm often causes the so-called "razor effect", in which the edges of the vascular catheter scrape against the wall of the artery. This is painful for the patient, can cause the artery to go into spasm and can cause snagging and serious bleeding complications.

Where spasm or snagging occurs, the procedure is frequently delayed while the patient is given a combination of sedative, painkilling (e.g. opiate) and artery dilating drugs. In some circumstances, the procedure has to be done via a different artery or even abandoned altogether. If the procedure is done via the femoral artery instead, there are additional risks of bleeding and major complication.

Whilst the prior art provides certain solutions to help reduce the risk of some of these problems arising, such solutions often require expensive devices. Furthermore, many prior art solutions are incompatible with standard equipment and are compatible only with specific catheter hardware, so multiple devices are required to enable an appropriate one to be selected for use with a particular catheter.

It is an object of certain embodiments of the present invention to overcome at least some disadvantages associated with the prior art.

Known examples of catheter systems include prior patent document US5976153A which describes an example of a a stent delivery catheter system for placing a stent within a stenosis in a vessel of a human body. The stent delivery catheter system utilizes a slideable sheath with a thin-walled distal portion that is situated coaxially over a stent that is placed onto a balloon located at the distal portion of a balloon angioplasty catheter. The distal end of a central portion of the sheath has an interior shoulder which is capable of exerting a distally directed push force on the balloon angioplasty catheter at a point that is just proximal to the stent. This push force is then transferred through the non-deployed stent to a gradually tapered, highly flexible, lubricity coated distal tip of the balloon angioplasty catheter. The structure of a continuous outer surface extending backward from the distal tip and onto the distal section of the sheath, plus the lubricity coating of the outer surfaces of the distal tip and the sheath, plus the increased stent delivery catheter system pushability provided by the sheath and the stent itself makes it possible for the stent delivery catheter system to be pushed through even very tight stenoses.

### BRIEF SUMMARY OF THE DISCLOSURE

The invention is defined by the appended claims. In accordance with a first aspect of the present invention there is provided a guiding device for a vascular catheter that is inserted over a guidewire, the guiding device comprises:
a tapered component for passing through a bore of the vascular catheter and extending from a distal end thereof,
the tapered component having an aperture through which the guidewire may pass so that the tapered component may move along the guidewire; and
pushing means extending from the tapered component and configured to extend beyond a proximal end of the vascular catheter;
wherein the tapered component comprises a first section, a second section and the third section; the first section is disposed between the proximal end of the tapered component and the second section; the second section is disposed between the first section and the third section; and the third section is disposed between the second section and a distal end of the tapered component;
wherein the first section is a cylinder, the second and the third sections are conical, and the taper of the second section differs from the taper of the third section.

In certain embodiments, the second section may be a convex conic section. The third section may be a concave conic section. In certain embodiments, the proximal end of the first section may coincide with the proximal end of the tapered component. The diameter of the first section may be the maximum diameter of the tapered component. In certain embodiments, the distal end of the third section may coincide with the distal end of the tapered component. The minimum diameter the tapered component may coincide with the distal end of the third section.

Movement of the tapered component along the guidewire may be controllable proximally from beyond the proximal end of the vascular catheter by movement of the pushing means.

The guiding device may comprise a connector, connectable to the pushing means, for releasably connecting the guiding device to the proximal end of the vascular catheter. The connector may comprise a free-wheeling mechanism configured to permit the connection of the connector to the proximal end of the vascular catheter without causing rotation the pushing means. In certain embodiments, the connector may comprise a body connectable to the pushing means and a free-wheeling ring for connecting to the catheter. The free-wheeling ring may be axially restrained relative to the body of the connector and may rotate freely about the body of the connector. A rim may restrain the free-wheeling ring in the distal direction relative to the body of the connector. The connector may be movable along the pushing means.

In certain embodiments, the pushing means may have a non-circular cross-section. The cross-section of the pushing means may have a plurality of sides. The cross-section of the pushing means may have four sides. Adjacent sides may be of different lengths. The corners between adjacent sides may be rounded. In certain embodiments, the plurality of sides may comprise a first curved side. The first curved side may conform with a part of the circumference of the outer surface of the proximal end of the tapered component.

In certain embodiments, the guiding device may comprise an internal bore accessible through the aperture. The plurality of sides of the pushing means may comprise a second curved side that conforms with a part of the circumference of the internal bore of the tapered component.

In certain embodiments, the pushing means may be arranged to permit a portion of the guidewire that is radially adjacent to the pushing means to remain visible to an operator in use.

In any of the first aspect of the invention, the tapered component may have a low friction outer surface. In certain embodiments, the low friction outer surface may comprise a hydrophilic outer surface.

In certain embodiments, the pushing means may comprise a wire extending from the tapered component.

In certain embodiments, the tapered component may have an axial length between 10 cm and 30 cm, and optionally between 14 cm and 25 cm. In certain embodiments, the tapered component may have an axial length of about 20 cm

The pushing means may optionally extend from the tapered component by at least 60 cm, at least 70 cm, at least 80 cm, at least 90 cm or at least 100 cm.

In certain embodiments, the aperture may be sized to permit the passage therethrough of a guidewire having a diameter of 0.46 mm (0.018"), 0.64 mm (0.025"), 0.81 mm (0.032"), 0.89 mm (0.035"), 0.97 mm (0.038") or 1.10 mm (0.043").

The tapered component may have a maximum diameter that is is less than 3.00 mm, less than 2.7 mm, less than 2.29 mm, less than 2.0 mm, less than 1.7 mm or less than 1.35 mm. In certain embodiments, the maximum outer diameter may be coincident with a proximal end of the tapered component, and the tapered component may taper from a minimum outer diameter at a distal end of the tapered component to the maximum outer diameter.

The guiding device may further comprise a radiopaque material. In certain embodiments, the tapered component may comprise a radiopaque material. In a non-limiting embodiment, the radiopaque material may be Barium Sulphate. In certain embodiments, the guiding device may further comprise one or more markers. The one or more markers may comprise one or more radio-opaque markers for indicating the location of a part of the guiding device under fluoroscopic screening. The one or more radio-opaque markers may be disposed on the tapered component. In alternative embodiments, the one or more markers may be provided on the pushing means, e.g. for indicating a deployed length of the guiding device. In certain embodiments, the guiding means may comprise ink or physical bands which make the distal end of the tapered component visible to x-rays and other medical imaging techniques.

In certain embodiments, the tapered component may comprise plastics material.

In certain embodiments, the pushing means may comprise metal and/or plastics material.

In accordance with another aspect of the present invention, there is provided a kit comprising:
a guiding device as described above; and
a vascular catheter;
wherein the tapered component of the guiding device is configured to pass through a bore of the vascular catheter and extend from a distal end thereof.

The kit may further comprise a guidewire, wherein the aperture of the tapered component may permit the passage of the guidewire therethrough so that the tapered component may move along the guidewire.

In accordance with another aspect of the present invention, there is provided a kit comprising:
a guiding device as described above;
a guidewire;
wherein the aperture of the tapered component may permit the passage of the guidewire therethrough so that the tapered component may move along the guidewire.

### BRIEF DESCRIPTION OF THE DRAWINGS

Embodiments of the invention are further described hereinafter with reference to the accompanying drawings, in which:
Figure 1A is a schematic side-view of a guiding device in accordance with an embodiment of the present invention;
Figure 1B is a schematic side-view of a guiding device in accordance with an embodiment of the present invention in use with a catheter;
Figure 2 is a schematic side-view of a part of a guiding device in accordance with an embodiment of the present invention;
Figure 3 is a schematic side-view of a part of a guiding device in accordance with an embodiment of the present invention in use with a catheter and guidewire;
Figure 4 is a schematic cross-section of a guiding device in accordance with an embodiment of the present invention;
Figure 5 is schematic cross-sections of a pushing means of a guiding device according to embodiments of the invention;
Figure 6 is a schematic cross-section of a distal end of a connector for a guiding device in accordance with an embodiment of the present invention;
Figure 7 is a schematic side-view of a connector for a guiding device in accordance with an embodiment of the present invention; and
Figure 8 is an exploded schematic side-view of a a connector for a guiding device in accordance with an embodiment of the present invention.

### DETAILED DESCRIPTION

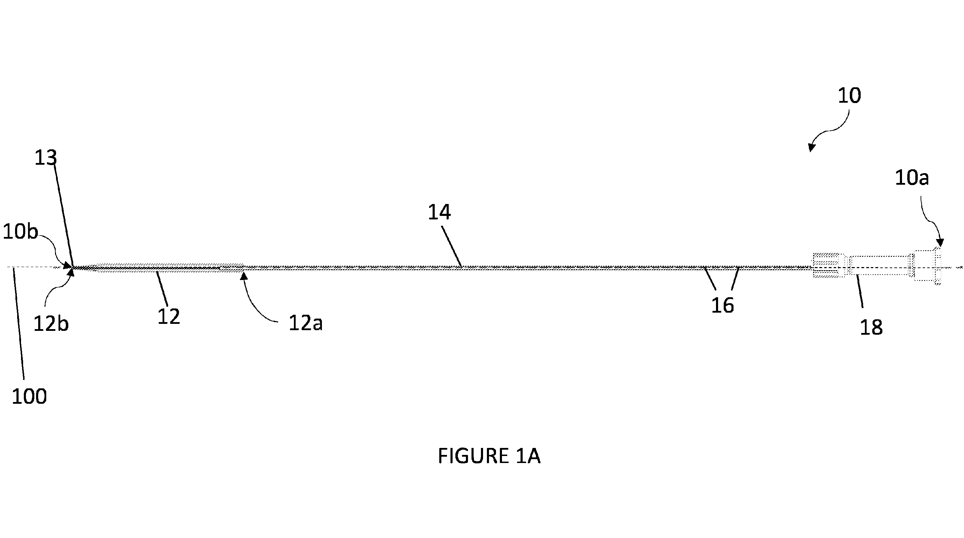

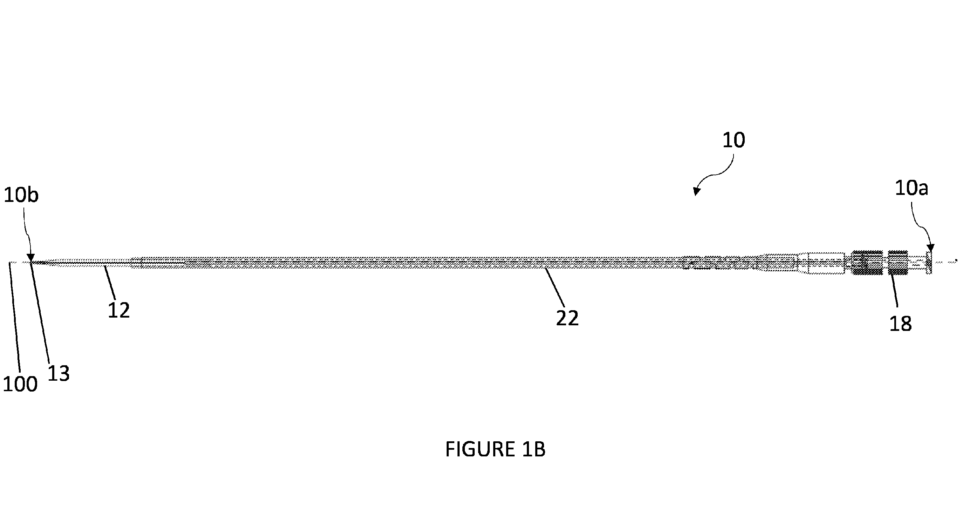

Figures 1A and 1B show schematic side-views of a guiding device 10 according to an embodiment of the present invention. The guiding device 10 may facilitate improved passage of a vascular catheter through a peripheral blood vessel (e.g. the radial artery).

The guiding device 10 extends along a longitudinal axis 100 between a proximal end 10a and a distal end 10b. Throughout the present specification, references to forward or distal directions (and similar) relate to directions towards the distal end 10b (or away from the proximal end 10a) that are parallel to the longitudinal axis 100. Similarly, references to rearward or proximal directions (and similar) relate to directions towards the proximal end 10a (or away from the distal end 10b) that are parallel to the longitudinal axis 100. References to axial directions and the like relate to directions that are parallel to the longitudinal axis 100. References to radial directions and the like relate to directions that extend orthogonally to the longitudinal axis 100, where a radially outward position is further from the longitudinal axis 100 than a radially inward position. References to circumferential directions and the like relate to paths that follow the circumference (either in part or completely) of a notional circle whose plane extends orthogonally to the longitudinal axis 100. References to cross-sections relate to cross-sections in a plane perpendicular to the longitudinal axis 100.

The guiding device 10 comprises a tapered component 12 that extends generally along the longitudinal axis 100 from a proximal end 12a to a distal end 12b. In the non-limiting embodiment shown in the Figures, the distal end 12b of the tapered component 12 is coincident with the distal end 10b of the guiding device 10. The tapered component 12 tapers (i.e. increases in diameter) from a minimum diameter at its distal end 12b to a larger diameter rearward of the distal end 12b. In certain embodiments, the maximum diameter may be coincident with the proximal end 12a of the tapered component 12.

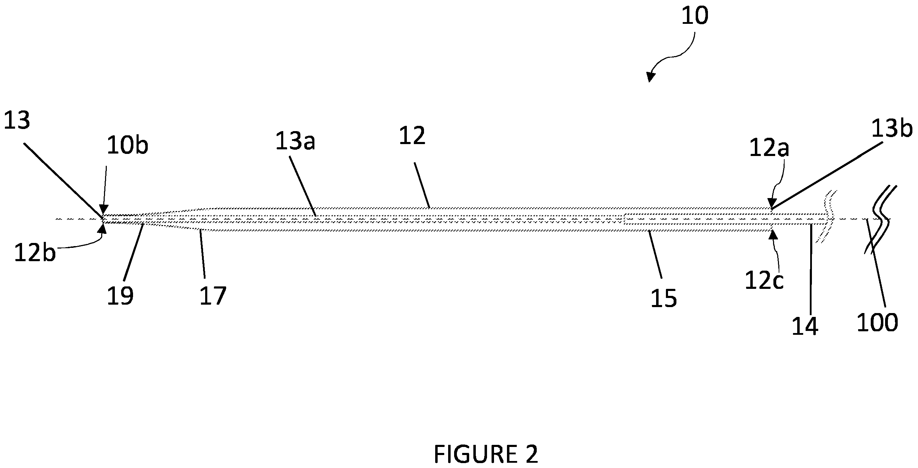

In certain embodiments, the shape of the taper changes along the tapered component 12 between the proximal end 12a and the distal end 12b. In the embodiment shown in Figure 2, the tapered component 12 comprises a first section 15, a second section 17 and a third section 19. Each section takes a different shape. However, the resulting outer profile of the tapered component 12 is continuous along a direction parallel to the longitudinal axis 100. The first section 15 is disposed between the proximal end 12a of the tapered component 12 and the second section 17. In the embodiment shown in Figure 2, the first section 15 takes the form of a cylinder. That is, the cross-section of the first section is substantially uniform along its length. The proximal end of the first section 15 may coincide with the proximal end 12a of the tapered component 12. In certain embodiments, the diameter of the first section 15 may be the maximum diameter of the tapered component 12. The second section 17 is disposed between the first section 15 and the third section 19. In the embodiment shown in Figure 2, the second section 17 takes the form of a convex cone. That is, the profile of the taper of the cone follows a convex curve. The diameter of the cross-section of a convex cone decreases at an increasing rate. The maximum diameter of the second section 17 coincides with the proximal end of the second section 17 and the diameter of the second section decreases in the distal direction. In certain embodiments, the diameter of the proximal end of the second section 17 may equal the diameter of the first section 15. The third section 19 is disposed between the second section 17 and the distal end 12b of the tapered component 12. In the embodiment shown in Figure 2, the third section takes the form of a concave cone. That is, the profile of the taper of the cone follows a concave curve. The diameter of the cross-section of a concave cone decreases at a decreasing rate. The maximum diameter of the third section 19 coincides with the proximal end of the third section and the diameter of the third section 19 decreases in the distal direction. In certain embodiments, the diameter of the proximal end of the third section 19 may equal the diameter of the distal end of the second section 17. The distal end of the third section 19 may coincide with the distal end 12a of the tapered component 12. In certain embodiments, the minimum diameter of the tapered component 12 may be the diameter of the distal end of the third section 19. The above-described shape of the tapered component 12 provides it with improved flexibility relative to the prior art, enabling it to navigate through blood vessels effectively. As a result, in certain embodiments, the tapered component 12 may be manufactured without the need to vary the materials or material properties (e.g. hardness) along its length.

In other embodiments, the tapered component may take any alternative shape capable of providing it with the required flexibility. In alternative embodiments, the tapered component may comprise a different number of sections. For example, a fourth section may extend distally from the third section. The fourth section (not shown) may take the form of a cylinder. Additionally or alternatively, a fifth section may extend proximally from the first section. The fifth section (not shown) may take the form of a cylinder. In certain embodiments, additional sections may be disposed between the first 15 and the second 17 sections and/or between the second 17 and third 19 sections. Additionally, the shape of each section may take a different form. The second section 17 may take the form of a cone (i.e. the profile of the taper of the cone follows a straight line). Alternatively, the second section 17 may take the form of a concave cone or the second section 17 may have a radiused or a parabolic taper. The third section 19 may take the form of a cone (i.e. the profile of the taper of the cone follows a straight line). Alternatively, the third section 19 may take the form of a convex cone or third section 19 may have a radiused or a parabolic taper. In certain embodiments, the taper of the second section 17 may differ from the taper of the third section 19. For example, the taper of the second section 17 may be steeper (at least in parts) than the taper of the third section 19 relative to the longitudinal axis 100.

The tapered component 12 includes an aperture 13 at its distal end 12b. The tapered component 12 is hollow such that an internal bore 13a of the tapered component 12 is accessible through the aperture 13. The internal bore 13a of the tapered component 12 extends to the proximal end 12a of the tapered component 12 so that it is also accessible from the proximal end 12a. Thus, the internal bore 13a extends through all sections of the tapered component 12. The internal bore 13a may have a constant diameter from the proximal end 12a to the distal end 12b of the tapered component 12. The internal bore 13a is accessible through a second aperture 13b in the proximal end 12a of the tapered component 12. A wall 12c may extend between the internal bore 13a and the outer surface of the proximal end 12a of the tapered component 12. In certain embodiments, at least a part of the wall 12c may be substantially perpendicular to the longitudinal axis 100. Additionally or alternatively, at least a part of the wall 12c may be inclined relative to the longitudinal axis 100. Thus, the wall 12c may provide a tapered opening for the second aperture 13b.

The guiding device 10 also comprises a pushing means 14 (e.g. a pusher in the form of a pushing component or assembly of components) that extends rearwardly from the tapered component 12. The pushing means 14 may additionally extend from a position on the tapered component 12 that is axially forwards of the proximal end 12a of the tapered component 12. The pushing means 14 may be inserted into a groove in the tapered component 12. The pushing means 14 is sufficiently rigid to move the tapered component 12 both forwards and backwards through a blood vessel, when the pushing means 14 extends proximally from the tapered component 12 out of the blood vessel (and out of the body). Despite such rigidity, the pushing means 14 is also sufficiently flexible so as to be capable of bending and following the contours of a blood vessel as it passes therethrough.

The guiding device 10 additionally includes a connector 18 that is connected to the pushing means 14. The connector 18 may facilitate easier handling and movement of the pushing means 14 from outside the blood vessel. The connector 18 may be configured to releasably connect the guiding device 10 to a proximal end of a vascular catheter 22.

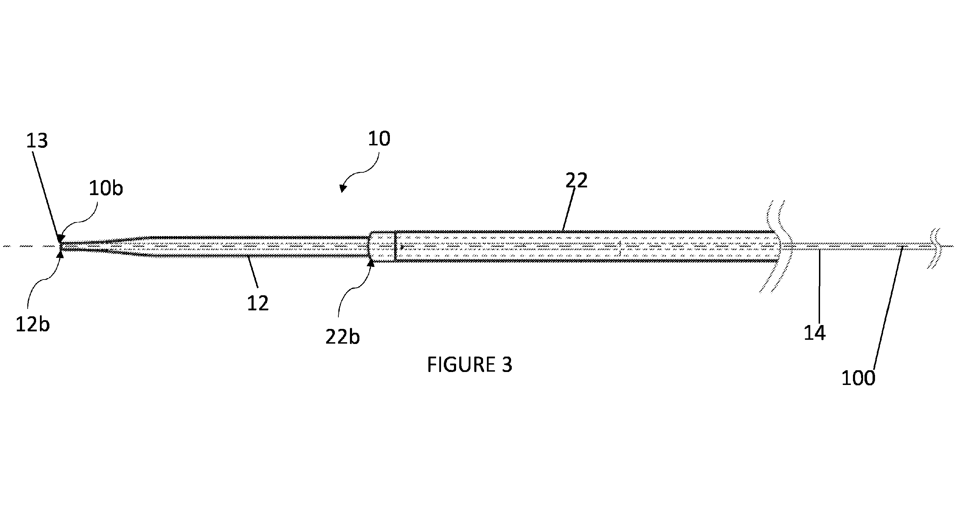

With reference to Figures 1B and 3, in use, the guiding device 10 is passed through a bore of a vascular catheter 22 so that at least a portion of the tapered component 12 extends from a distal end 22b of the vascular catheter 22. In the schematic side-view of Figures 1B and 3, the vascular catheter 22 is shown as translucent to aid understanding of the depicted embodiment of the invention. The depicted length of the vascular catheter relative to the length of the tapered component is shortened in the Figures to facilitate understanding of the embodiment. The term vascular catheter refers to any suitable catheter for use in a blood vessel (i.e. an artery or vein). The tapered component 12 and the vascular catheter 22 are threaded over a guidewire (not shown) such that the guidewire passes through the aperture 13 of the tapered component 12, through the internal bore 13a of the tapered component 12, and through the bore of the vascular catheter 22. In such a configuration, the guidewire extends forwardly from the distal end 12b of the tapered component 12 and rearwardly from the proximal end (not shown) of the vascular catheter 22.

Rearwardly of the proximal end of the vascular catheter 22, the guidewire and the pushing means 14 extend alongside one another. As such, axial movement of the pushing means 14 causes the axial movement of the tapered component 12 along the guidewire 20. In use the guidewire may be disposed within a blood vessel such that it may guide passage of the vascular catheter 22, while the proximal end of the vascular catheter 22 remains outside of the blood vessel. Given that the tapered component 12 extends beyond the distal end 22b of the vascular catheter 22, the tapered component 12 leads the vascular catheter 22 in the blood vessel. Thus, the passage of the vascular catheter 22 in the blood vessel is made easier by the tapered component 12 thereby substantially avoiding the "razor effect" of the front edge of the proximal end of the catheter 22 in its interaction with the lining of the blood vessel. Consequently, the risk of the vascular catheter 22 snagging the walls of the blood vessel is reduced, as is the risk of the blood vessel going into spasm. As a result, patient comfort may be improved. These advantages may serve to increase the success rate of procedures (such as coronary angioplasty or percutaneous intervention (PCI)) in blood vessels such as the radial and brachial arteries (in which problems associated with the "razor effect" are more prevalent), improving their viability over procedures in other blood vessels such as the femoral artery. In preferable embodiments, the tapered component 12 is always partly contained within the vascular catheter 22 whilst the guiding device 10 is guiding the passage of the vascular catheter 22 through a blood vessel. In certain embodiments, the first portion 15 of the tapered component 12 may always be contained within the vascular catheter 22.

Notably, the pushing means 14 does not extend so as to be coaxial with the longitudinal axis 100, whereas the guidewire extends substantially along the longitudinal axis 100. Consequently, the pushing means 14 does not interfere with the path of the guidewire 20. Importantly, the pushing means 14 is configured such that a portion of the guidewire that is radially adjacent to the pushing means 14 remains visible to an operator in use (i.e. when using the pushing means 14 to move the tapered component 12 along the guidewire 20). Therefore, the operator may maintain the guidewire in sight to ensure that the whole guidewire does not inadvertently advance into the blood vessel.

In the preferable embodiment shown in the Figures, the pushing means 14 comprises a wire that has a stiffness that permits the tapered component 12 to be advanced in the blood vessel by pushing on the wire. In alternative embodiments, the pushing means 14 may take any alternative form that is capable of advancing the tapered component 12 and that permits a portion of the guidewire that is radially adjacent to the pushing means 14 to remain visible to an operator in use. For example, the pushing means may comprise a rod. A wire or rod is only radially aligned with the guidewire over a small angular extent, and so provides sufficient means to push (and pull) the tapered component 12 without significantly visually obscuring the exposed guidewire 20. Similarly, other preferable variations of the pushing means 14 may be radially aligned with the guidewire over a small angular extent (e.g. less than 180°, less than 90°, less than 45°, less than 20°, less than 10° or less than 5°). The pushing means 14 may be made of any suitable material that is sufficiently rigid to facilitate the advancement of the tapered component 12 in the blood vessel by manipulation of the pushing means 14. In certain embodiments, the pushing means 14 may be made of a metal (e.g. if the pushing means 14 is a wire or rod).

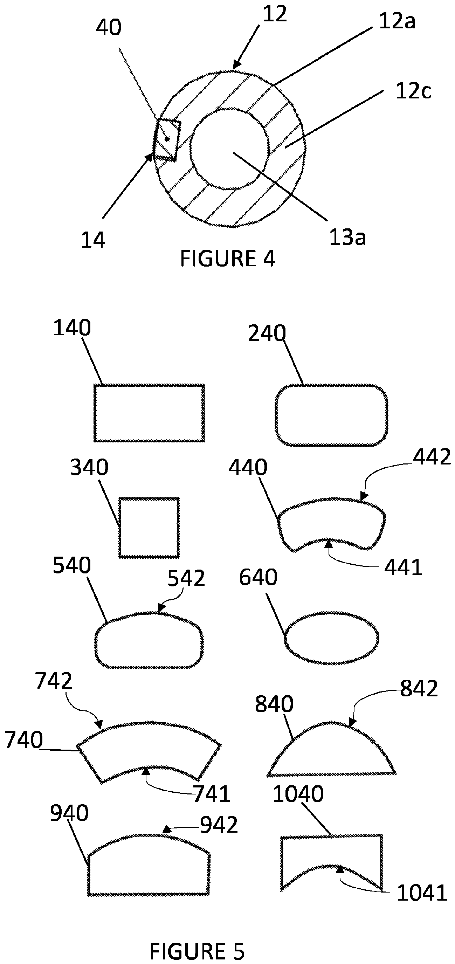

Figure 4 shows a cross-section of an embodiment of the guiding device 10 at the proximal end 12a of the tapered component 12. In the embodiment shown in Figure 4, the distal end of the pushing means 14 extends from the wall 12c. As described above, the wall 12c extends between the inner bore 13a and the outer surface of the proximal end 12a of the tapered component 12. The wall 12c has a limited thickness. For example, in a 6F size catheter the tapered component has a maximum diameter of less than 1.7 mm and an inner bore diameter of about 1.1 mm, thus, providing a wall with a thickness of about 0.3 mm and from which the pushing means extends. In certain embodiments, the cross-section 40 of the pushing means 14 is non-circular to facilitate the connection of the pushing means 14 to the tapered component 12. In certain embodiments, the non-circular cross-section 40 is substantially across the length of the pushing means 14. For example, the non-circular cross-section 40 may be across more than 50%, 60%, 70%, 80% or 90% of the length of the pushing means 14. In alternative embodiments, the cross-section may vary along the length of the pushing means 14. A non-circular cross-section 40 improves the attachment of the pushing means 14 to the wall 12c that has a limited thickness. Pushing means 14 with non-circular cross-sections 40 have different bending properties along difference axes of the cross-sectional profile. However, since the pushing means 14 is long (relative to its width) and, in use, constrained within the lumen of the guide catheter, the impact of the different bending properties of the pushing means 14 on the control of the tapered component 12 are minimal and within acceptable levels. In certain embodiments, the non-circular cross-section 40 provides the pushing means 14 with extra pushability whilst maintaining flexibility and a small cross-sectional profile of the pushing means 14.

The pushing means 14 may take any non-circular cross-section 40 suitable for attachment to the tapered component 12 that can provide the required rigidity and flexibility to advance the tapered component 12 along the guidewire 20. The pushing means 14 may also take a non-circular cross-section 40 that ensures that the guidewire remains visible during use. The cross-section 40 of the pushing means 14 may comprise a plurality of sides. For example, the cross-section 40 may have four sides. One or more of the plurality of sides may be a curved side. The one or more curved sides may be a convex or a concave curve. Additionally, the cross-section may have a convex curved side and a concave curved side. In certain embodiments, the cross-section 40 may comprise a curved side that, for example, conforms with a part of the circumference of the internal bore 13a. Alternatively or additionally, the cross-section 40 may comprise a curved side that conforms with a part of the circumference of the outer surface of the proximal end 12a of the tapered component 12. The plurality of sides of the cross-section 40 may comprise adjacent sides having different lengths. In certain embodiments, the cross-section 40 of the pushing means 14 may have a first side with a length between 0.40 mm to 0.53 mm and a second side, adjacent to the first side, with a length between 0.20 mm to 0.30 mm. In certain embodiments, there may be well defined corners between adjacent sides. Alternatively, the corners between adjacent sides of the profile may be rounded. The angle between a pair of adjacent sides may be 90°, greater than 90° or less than 90°.

Figure 5 shows examples of the cross-sectional profile of the pushing means 14 according to embodiments of the invention. However, the cross-section 40 of the pushing means 14 is not limited to the embodiments shown in Figure 5. Additionally, in certain embodiments, the cross-section 40 of the pushing means 14 may vary along the length of the pushing means 14. The cross-section 40 of the pushing means 14 may be substantially a rectangle 140, 240, a square 340 or an annular arc 440, 740. In certain embodiments, the rectangle, the square or the annular arc may have rounded corners 240, 440. The annular arc 440, 740 may comprise a curved side 441, 741 that conforms with a part of the circumference of the internal bore 13a of the tapered component 12. Additionally or alternatively, the annular arc 40d, 40g may comprise a curved side 442, 742 that conforms with a part of the circumference of the outer surface of the proximal end 12a of the tapered component 12. In certain embodiments, the cross-section 40 of the pushing means 14 may be substantially an ellipse or an oval 640. In alternative embodiments, the cross-section 40 of the pushing means 14 may comprise a circular segment 840. The curved side 842 of the circular segment 840 may conform with a part of the circumference of the outer surface of the proximal end 12a of the tapered component 12. In certain embodiments, the cross-section 40 may be a semi-circular. In certain embodiments, the cross-section 40 of pushing means 14 may be an oblong-like shape 540, 940, 1040 with at least one curved side 542, 942, 1041. The curved side 542, 942 may conform with a part of the circumference of the outer surface of the proximal end 12a of the tapered component 12. The curved side 1041 may conform with a part of the circumference of the internal bore 13a of the tapered component 12. In certain embodiments, the oblong-like shape may comprise a first and a second side where a first end each of the first and second sides are joined at right angles to opposite ends of a third side. The third side may be longer than the first and second sides. The second ends of the first and second sides may be joined to opposite ends of a fourth side where the fourth side is a curved side. The fourth side may be a convex or a concave curve.

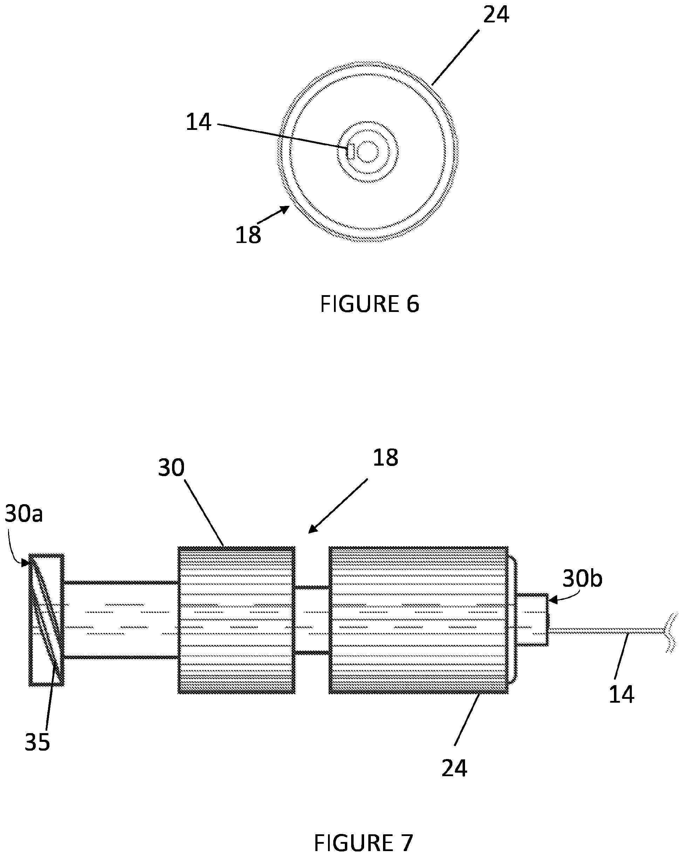

Once the pushing means 14 has been used to advance the tapered component 12 into the vascular catheter 22 so that the tapered component 12 extends beyond the distal end 22b of the vascular catheter 22, the connector 18 may releasably connect the guiding device 10 to the vascular catheter 22. Figures 6 and 7 show a connector 18 according to an embodiment of the invention. By connecting the guiding device 10 to the vascular catheter 22, over-insertion of the guiding device 10 in the vascular catheter 22 may be prevented. In particular, the connection between the guiding device 10 and the vascular catheter 22 will determine the maximum extent to which the tapered component 12 extends out of the distal end 22b of the vascular catheter 22. Additionally, the connection between the guiding device 10 and the vascular catheter 22 will cause the guiding device 10 and the vascular catheter to move collectively as one (i.e. with minimal relative movement therebetween) along the guidewire 20.

In certain embodiments, the connector 18 may be located at or towards the proximal end 10a of the guiding device 10. The connector 18 may be connected to the pushing means 14 rearward of the proximal end 12a of the tapered component 12. In certain embodiments, the connector 18 may be moveable along the pushing means so as to be engageable with vascular catheters 22 of various lengths.

The connector 18 may releasably connect the guiding device 10 to the proximal end of the vascular catheter 22 by any suitable fixing mechanism, including but not limited to push fit and snap fit arrangements. In certain embodiments, the connector may be rotatably connectable to the vascular catheter 22. In such embodiments, the connector 18 comprises a free-wheeling mechanism. The free-wheeling mechanism is configured to permit connection of the connector 18 to the proximal end of the vascular catheter 22 without causing rotation of the pushing means 14. Therefore, when rotating the connector 18 to connect it to the vascular catheter 22, the tapered component 12 is not rotated within the vascular catheter 22 or the blood vessel.

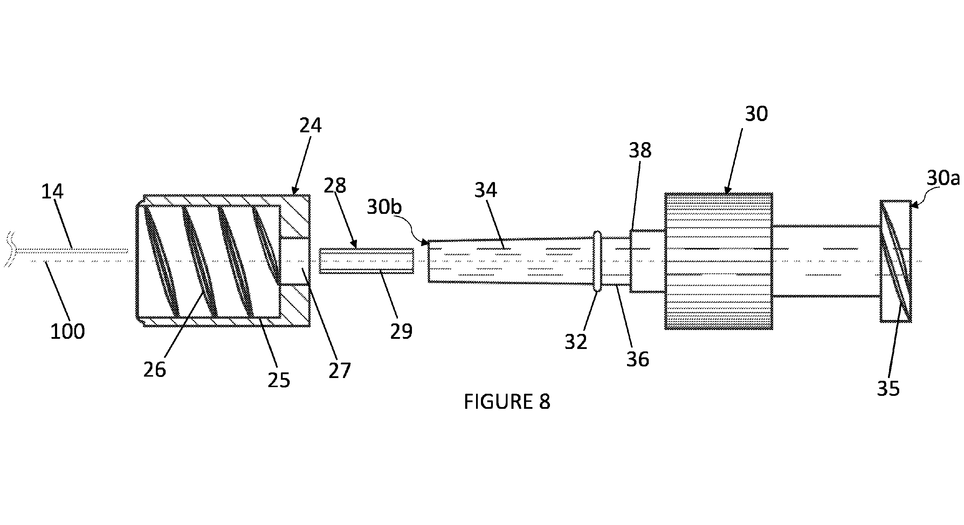

Figures 6 to 8 show a non-limiting embodiment of the connector 18 according the invention. Figures 6 and 7 show the connector 18 in the assembled state and connected to the pushing means 14 whilst Figure 8 shows an exploded side-view of the connector 18. The connector 18 comprises a body 30, a locking tube 38 and a free-wheeling ring 24. When assembled, the body 30 and free-wheeling ring 24 form the free-wheeling mechanism.

The body 30 comprises a bore 34 which extends through the body 30 from the proximal end 30a to the distal end 30b. The central axis of the bore 34 coincides with the longitudinal axis 100 of the guiding device 10. The body 30 has an annular rim 32. The rim 32 may be proximally spaced from the distal end 30b of the body 30. In alternative embodiments, the rim may be at the distal end 30b of the body 30. The rim 32 may be continuous or may comprise a plurality of separate raised sections. The body 30 comprises a shoulder 38 proximally spaced from the rim 32. The shoulder 38 extends circumferentially around the body 30. The shoulder 38 may be continuous or may comprise a plurality of separate raised sections. A channel 36 is formed between the rim 32 and shoulder 38. The channel 38 is configured to receive a portion of the free-wheeling ring 24. In certain embodiments, the proximal end 30a of the body 30 may comprise a thread 35 for connecting the body 30 to additional components. In certain embodiments, the body 30 may be a single injection moulding.

The free-wheeling ring 24 comprises an internal cavity 25 at the distal end of the ring 24. The internal cavity 25 comprises a thread 26 for connecting to the proximal end of the catheter 22. In certain embodiments, the internal cavity 25 may comprise a female luer thread. The free-wheeling ring 24 comprises an aperture 27 at the proximal end of the ring 24. The aperture extends through the proximal wall of the free-wheeling ring 24 into the internal cavity 25. The aperture 27 is configured to receive the proximal end 30b of the body 30. The diameter of the aperture 27 is configured to form a snap-fit over the annular rim 32 and reside between the annular rim 32 and the shoulder 38. The diameter of the aperture 27 is smaller than the diameter of the rim 32 and the diameter of the shoulder 38.

During assembly of the connector 18, the proximal end 30b of the body 30 is inserted through the aperture 27 of the free-wheeling ring 24. The aperture 27 of the free-wheeling ring 24 is pushed over the rim 32 so that the free-wheeling ring 24 is retained within the channel 36. Therefore, in the assembled state, the free-wheeling ring 24 is axially restrained relative to the body 30 by the rim 32 and the shoulder 38 but can freely rotate around the body 30. The rotation axis of the free-wheeling ring 24 may be the longitudinal axis 100.

The pushing means 14 is connected to the body 30 by the locking tube 28. The outer diameter of the locking tube 28 is smaller than the diameter of the bore 34 of the body 30. The inner diameter of the locking tube 28 is greater than the diameter of the guide wire (not shown).

During connection of the pushing means 14 to the body 30, the pushing means 14 is inserted into the bore 34 of the body 30. The locking tube 28 is also inserted into the bore 34 of the body 30 with an adhesive to hold the locking tube 28 and the pushing means 14 securely within the body 30. The locking tube 28 holds the pushing means 14 at a radially offset position relative to longitudinal axis 100. This corresponds to the pushing means 14 extending from the tapered component 12 at a position radially offset form the longitudinal axis 100.

Once the connector 18 has been assembled and the pushing means 14 connected to the body 30, the free-wheeling ring 24 can be rotated to connect the connector 18 to the proximal end of the vascular catheter 22. Since the free-wheeling ring 24 rotates relative to the body 30, the connection to the vascular catheter 22 does not cause the pushing means 14 to rotate. Consequently, the tapered component 12 is not rotated within the vascular catheter 22 or the blood vessel when the guiding device 10 is connected to the catheter. The connector 18, therefore, provides a secure connection between the guiding device 10 and the vascular catheter 22 whilst avoiding rotation of the tapered component 12.

Once the vascular catheter 22 has been advanced to the desired position within the blood vessel, the connector 18 may be disconnected from the vascular catheter 22 and the pushing means 14 may be pulled rearwardly to withdraw the guiding device 10 from the blood vessel along the guidewire 20, leaving the vascular catheter 22 in place. Advantageously, the guiding device 10 may be used to reduce the risk of the artery spasming during the standard insertion procedure. This contrasts with certain prior art methods in which the standard guidewire must be removed and additional components inserted in order to continue advancement of a catheter once a restricted, tortuous or spastic artery is encountered.

In addition to assisting with the guiding of the vascular catheter 22 in the blood vessel ahead of the advancing catheter, the guiding device 10 may be used to "rescue" a procedure where snagging or spasm are encountered and the passage of the vascular catheter 22 along the guidewire in the blood vessel may have become obstructed and further advancement of the vascular catheter 22 may be inhibited or prevented. In such a situation, the guiding device 10 may be advanced along the guidewire and through the bore of the vascular catheter 22 so that the tapered component 12 protrudes from the distal end 22b of the vascular catheter 22. The tapered component 12 may then dilate the blood vessel to facilitate onward advancement of the vascular catheter 22 in the blood vessel and/or reducing the pain and discomfort experienced by the patient associated with this. Additionally, guiding devices according to certain embodiments of the invention may be used via a sheath or without a sheath, as the device may be used as an introducer for the guiding catheter and the combination may be known as a sheathless catheter and may be universal, in that it may be used with any manufacturer's catheters and guidewires.

In certain preferable embodiments, the tapered component 12 may have a low friction outer surface. A low friction interface with the blood vessel wall facilitates smoother travel of the tapered component in the blood vessel. In certain embodiments, any lubricious and/or hydrophilic outer surface may provide such a low friction outer surface. In certain embodiments, the hydrophilic outer surface may be provided by a hydrophilic coating that is applied to the tapered component 12. In alternative embodiments, the hydrophilic outer surface may be provided by the surface topography of the tapered component 12 (e.g. a plurality of grooves, recesses or other surface features that may lend the tapered component 12 hydrophilic properties).

The tapered component 12 may be made of any suitable material. In certain embodiments, the tapered component 12 comprises plastics material. Additionally or alternatively, the tapered component 12 may comprise a resilient material that may be deformable in use within the blood vessel. The tapered component 12 may be formed from a single material.

In certain embodiments, the tapered component 14 has an axial length of up to 30 cm. In certain embodiments, the tapered component 14 may have an axial length between 1 cm and 30 cm, or between 14 cm and 25 cm. In certain embodiments, the tapered component 14 has an axial length of about 14 cm. The tapered component 14 may have any axial length suitable for providing it with the required flexibility.

When the guiding device 10 is for use with a 6F catheter, the tapered component 12 may have a axial length of 14 cm, with the cylindrical first section 15 having an axial length of 10 cm and an axial length of 4 cm between the distal end 12b of the tapered component and the cylindrical first section 15. The second section 17 may be a convex cone where the taper of the cone conforms to an arc of a circle with a radius between 2000 mm and 4000 mm. Additionally or alternative, the third section 19 may be a concave cone where the taper of the cone conforms an arc of a circle with a radius between 2000 mm and 4000 mm.

In certain embodiments, the maximum diameter of the tapered component 12 (e.g. in an uncompressed state) is less than the internal diameter of the catheter in which it is to be inserted. The tapered component may have a maximum diameter that is less than 3.00 mm, less than 2.7 mm, less than 2.29 mm, less than 2.0 mm, less than 1.7 mm or less than 1.35 mm. For example, for an 8F catheter, the maximum diameter may be less than 2.29 mm, for a 7F catheter, the maximum diameter may be less than 2.0 mm, or for a 6F catheter, the maximum diameter may be less than 1.7 mm. The skilled reader will appreciate that, in this context, "F" refers to the French Gauge system, where 1F is equivalent to a diameter of 1.3 mm.

In order to be compatible with a range of guidewires 20, in certain embodiments the apertures 13 and 29 may be sized to permit the passage therethrough of a guidewire having a diameter between about 0.5 mm and 1 mm, e.g. having a diameter of 0.46 mm (0.018"), 0.64 mm (0.025"), 0.81 mm (0.032"), 0.89 mm (0.035"), 0.97 mm (0.038") or 1.10 mm (0.043"). In certain non-limiting embodiments, the aperture 13 may be larger than 0.36 mm (0.014") as to permit the tapered component 12 to pass over a guidewire (e.g. an angioplasty wire) having a diameter of 0.36 mm (0.014").

The guiding device 10 may be dimensionally compatible with and be otherwise suitable for use with a range of known vascular catheters 22 and/or guidewires 20. It is preferable for a guiding device 10 of a single size to be usable with a range of vascular catheters 22 of different lengths. In order to be dimensionally compatible with a particular vascular catheter 22, the pushing means 14 must extend rearwardly beyond the proximal end of the vascular catheter 22 when the tapered component 12 is in a position in which it protrudes from the distal end 22b of the vascular catheter 22. In certain embodiments, the pushing means 14 may extend from the tapered component 12 by at least 60 cm, at least 70 cm, at least 80 cm, at least 90 cm or at least 100 cm. In certain embodiments, the pushing means 14 may be provided with one or more markers 16 (see Figure 1) for indicating a deployed length of the guiding device 10. For example, once a particular marker 16 is proximate to the proximal end of the catheter 22, the length by which the guiding device 10 (and hence vascular catheter 22) has been deployed can be deduced from the marker 16. In certain non-limiting embodiments, markers may comprise visual markers and/or non-visual markers, such as radio-opaque markers (e.g. tungsten or barium). In certain embodiments, one or more of the markers may be provided at any suitable location on the guiding device 10, not limited to the pushing means 14. A radio-opaque marker approximately 2 mm from the distal tip may indicate the location of the distal end of the device underfluoroscopic screening.

The present invention is defined in the appended claims. For the avoidance of doubt, the invention is not necessarily limited to the specific features and dimensions discussed above. The skilled reader will also appreciate that the features (and dimensions) discussed above are not exhaustive, and embodiments of the invention may be realized that have different features and/or dimensions.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise. The word "component" is not limited to monolithic features and, in certain embodiments, may relate to multi-piece arrangements.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments.

The reader's attention is directed to all papers and documents which are filed concurrently with or previous to this specification in connection with this application and which are open to public inspection with this specification.

## Claims

1. A guiding device (10) for a vascular catheter (22) that is inserted over a guidewire, the guiding device comprising:
a tapered component (12) for passing through a bore of the vascular catheter (22) and extending from a distal end (22b) thereof,
the tapered component (12) having an aperture (13) through which the guidewire may pass so that the tapered component (12) may move along the guidewire; and
pushing means (14) extending from the tapered component (12) and configured to extend beyond a proximal end of the vascular catheter (22);
wherein the tapered component (12) comprises a first section (15), a second section (17) and the third section (19); the first section (15) is disposed between the proximal end (12a) of the tapered component and the second section (17); the second section (17) is disposed between the first section (15) and the third section (19); and the third section (19) is disposed between the second section (17) and a distal end (12b) of the tapered component (12);
charaterised in that the first section (15) is a cylinder, the second and the third sections (17, 19) are conical, and the taper of the second section (17) differs from the taper of the third section (19).

2. A guiding device (10) according to claim 1, wherein the second section (17) is a convex conic section and/or wherein the third section (19) is a concave conic section.

3. A guiding device (10) according to any preceding claim, wherein the proximal end of the first section (15) coincides with the proximal end (12a) of the tapered component (12) and optionally wherein the diameter of the first section (15) is the maximum diameter of the tapered component (12).

4. A guiding device (10) according to any preceding claim, wherein the distal end of the third section (19) coincides with the distal end (12b) of the tapered component (12) and optionally wherein the minimum diameter the tapered component (12) coincides with the distal end of the third section (19).

5. A guiding device (10) according to any preceding claim, wherein movement of the tapered component (12) along the guidewire is controllable proximally from beyond the proximal end of the vascular catheter (22) by movement of the pushing means (14).

6. A guiding device (10) according to claim 5, comprising a connector (18), connectable to the pushing means (14), for releasably connecting the guiding device (10) to the proximal end of the vascular catheter (22), and optionally wherein the connector (18) is moveable along the pushing means (14).

7. A guiding device (10) according to claim 6, wherein the connector (18) comprises a free-wheeling mechanism configured to permit the connection of the connector (18) to the proximal end of the vascular catheter (22) without causing rotation the pushing means (14), and optionally wherein the connector (18) comprises a body (30) connectable to the pushing means (14) and a free-wheeling ring (24) for connecting to the catheter (22).

8. A guiding device (10) according to any of claims 5 to 7, wherein the pushing means (14) has a non-circular cross-section.

9. A guiding device (10) according to claim 8, wherein the cross-section of the pushing means (14) has a plurality of sides and, optionally, wherein the cross-section (40) of the pushing means (14) has four sides.

10. A guiding device (10) according to claim 9, wherein adjacent sides are of different lengths and/or wherein the corners between adjacent sides are rounded.

11. A guiding device (10) according to any of claims 9 to 10, wherein the plurality of sides comprises a first curved side (442; 542; 742; 842; 942), and optionally wherein the first curved side (442; 542; 742; 842; 942) conforms with a part of the circumference of the outer surface of the proximal end (12a) of the tapered component (12).

12. A guiding device (10) according to any preceding claim, comprising an internal bore (13a) accessible through the aperture (13).

13. A guiding device (10) according to claim 12, when dependent on claim 9, wherein the plurality of sides comprises a second curved side (441; 741; 1041) that conforms with a part of the circumference of the internal bore (13a) of the tapered component (12).

14. A guiding device (10) according to any preceding claim, wherein the pushing means (14) is arranged to permit a portion of the guidewire that is radially adjacent to the pushing means (14) to remain visible to an operator in use.

15. A kit comprising:
a guiding device (10) according to any preceding claim; and
at least one of:
(i) a vascular catheter (22) wherein the tapered component (12) of the guiding device (10) is configured to pass through a bore of the vascular catheter (22) and extend from a distal end (22a) thereof; and
(ii) a guidewire wherein the aperture (13) of the tapered component (12) may permit the passage of the guidewire therethrough so that the tapered component (12) may move along the guidewire.

## Patentansprüche

1. Leitvorrichtung (10) für einen Gefäßkatheter (22), der über einem Führungsdraht eingeführt wird, wobei die Leitvorrichtung umfasst:
eine konische Komponente (12) zum Verlaufen durch eine Bohrung des Gefäßkatheters (22) und mit Erstreckung von einem distalen Ende (22b) davon,
wobei die konische Komponente (12) eine Apertur (13) aufweist, durch die der Führungsdraht verlaufen kann, sodass sich die konische Komponente (12) entlang des Führungsdrahts bewegen kann; und
Schiebemittel (14), das sich von der konischen Komponente (12) erstreckt und dazu konfiguriert ist, sich über ein proximales Ende des Gefäßkatheters (22) hinaus zu erstrecken;
wobei die konische Komponente (12) einen ersten Abschnitt (15), einen zweiten Abschnitt (17) und den dritten Abschnitt (19) umfasst; der erste Abschnitt (15) zwischen dem proximalen Ende (12a) der konischen Komponente und dem zweiten Abschnitt (17) angeordnet ist; der zweite Abschnitt (17) zwischen dem ersten Abschnitt (15) und dem dritten Abschnitt (19) angeordnet ist;
und der dritte Abschnitt (19) zwischen dem zweiten Abschnitt (17) und einem distalen Ende (12b) der konischen Komponente (12) angeordnet ist;
**dadurch gekennzeichnet, dass** der erste Abschnitt (15) ein Zylinder ist, der zweite und der dritte Abschnitt (17, 19) konisch sind und der Konus des zweiten Abschnitts (17) sich von dem Konus des dritten Abschnitts (19) unterscheidet.

2. Leitvorrichtung (10) nach Anspruch 1, wobei der zweite Abschnitt (17) ein konvexer konischer Abschnitt ist und/oder wobei der dritte Abschnitt (19) ein konkaver konischer Abschnitt ist.

3. Leitvorrichtung (10) nach einem vorhergehenden Anspruch, wobei das proximale Ende des ersten Abschnitts (15) mit dem proximalen Ende (12a) der konischen Komponente (12) übereinstimmt, und optional, wobei der Durchmesser des ersten Abschnitts (15) der maximale Durchmesser der konischen Komponente (12) ist.

4. Leitvorrichtung (10) nach einem vorhergehenden Anspruch, wobei das distale Ende des dritten Abschnitts (19) mit dem distalen Ende (12b) der konischen Komponente (12) übereinstimmt, und optional, wobei der minimale Durchmesser der konischen Komponente (12) mit dem distalen Ende des dritten Abschnitts (19) übereinstimmt.

5. Leitvorrichtung (10) nach einem vorhergehenden Anspruch, wobei eine Bewegung der konischen Komponente (12) entlang des Führungsdrahts proximal von jenseits des proximalen Endes des Gefäßkatheters (22) durch Bewegung des Schiebemittels (14) steuerbar ist.

6. Leitvorrichtung (10) nach Anspruch 5, umfassend einen Verbinder (18), der mit dem Schiebemittel (14) verbindbar ist, zum lösbaren Verbinden der Leitvorrichtung (10) mit dem proximalen Ende des Gefäßkatheters (22), und optional, wobei der Verbinder (18) entlang des Schiebemittels (14) bewegbar ist.

7. Leitvorrichtung (10) nach Anspruch 6, wobei der Verbinder (18) einen Freilaufmechanismus umfasst, der dazu konfiguriert ist, die Verbindung des Verbinders (18) mit dem proximalen Ende des Gefäßkatheters (22) zu gestatten, ohne eine Drehung des Schiebemittels (14) zu verursachen, und optional, wobei der Verbinder (18) einen Körper (30), der mit dem Schiebemittel (14) verbindbar ist, und einen Freilaufring (24) zum Verbinden mit dem Katheter (22) umfasst.

8. Leitvorrichtung (10) nach einem der Ansprüche 5 bis 7, wobei das Schiebemittel (14) einen nicht kreisförmigen Querschnitt aufweist.

9. Leitvorrichtung (10) nach Anspruch 8, wobei der Querschnitt des Schiebemittels (14) eine Vielzahl von Seiten aufweist, und optional, wobei der Querschnitt (40) des Schiebemittels (14) vier Seiten aufweist.

10. Leitvorrichtung (10) nach Anspruch 9, wobei benachbarte Seiten verschiedene Längen aufweisen und/oder wobei die Ecken zwischen benachbarten Seiten abgerundet sind.

11. Leitvorrichtung (10) nach einem der Ansprüche 9 bis 10, wobei die Vielzahl von Seiten eine erste gekrümmte Seite (442; 542; 742; 842; 942) umfasst, und optional, wobei die erste gekrümmte Seite (442; 542; 742; 842; 942) einem Teil des Umfangs der Außenfläche des proximalen Endes (12a) der konischen Komponente (12) entspricht.

12. Leitvorrichtung (10) nach einem vorhergehenden Anspruch, umfassend eine durch die Apertur (13) zugängliche Innenbohrung (13a).

13. Leitvorrichtung (10) nach Anspruch 12, wenn abhängig von Anspruch 9, wobei die Vielzahl von Seiten eine zweite gekrümmte Seite (441; 741; 1041) umfasst, die einem Teil des Umfangs der Innenbohrung (13a) der konischen Komponente (12) entspricht.

14. Leitvorrichtung (10) nach einem vorhergehenden Anspruch, wobei das Schiebemittel (14) angeordnet ist, um einem Abschnitt des Führungsdrahts, der radial neben dem Schiebemittel (14) ist, zu gestatten, bei Verwendung für einen Bediener sichtbar zu bleiben.

15. Kit, umfassend:
eine Leitvorrichtung (10) nach einem vorhergehenden Anspruch; und
mindestens eines von:
(i) einem Gefäßkatheter (22) wobei die konische Komponente (12) der Leitvorrichtung (10) dazu konfiguriert ist, durch eine Bohrung des Gefäßkatheters (22) zu verlaufen und sich von einem distalen Ende (22a) davon zu erstrecken; und
(ii) einem Führungsdraht, wobei die Apertur (13) der konischen Komponente (12) den Durchgang des Führungsdrahts dort hindurch gestatten kann, sodass sich die konische Komponente (12) entlang des Führungsdrahts bewegen kann.

## Revendications

1. Dispositif de guidage (10) pour un cathéter vasculaire (22) qui est inséré sur un fil-guide, le dispositif de guidage comprenant :
un composant effilé (12) destiné à traverser un alésage du cathéter vasculaire (22) et s'étendant à partir d'une extrémité distale (22b) de celui-ci,
le composant effilé (12) comportant une ouverture (13) à travers laquelle le fil-guide peut passer de sorte que le composant effilé (12) puisse se déplacer le long du fil-guide ; et
un moyen de poussée (14) s'étendant à partir du composant effilé (12) et conçu pour s'étendre au-delà d'une extrémité proximale du cathéter vasculaire (22) ;
ledit composant effilé (12) comprenant une première section (15), une deuxième section (17) et la troisième section (19); ladite première section (15) étant disposée entre l'extrémité proximale (12a) du composant effilé et la deuxième section (17) ; ladite deuxième section (17) étant disposée entre la première section (15) et la troisième section (19) ; et ladite troisième section (19) étant disposée entre la deuxième section (17) et une extrémité distale (12b) du composant effilé (12) ;
**caractérisé en ce que** la première section (15) est un cylindre, les deuxième et troisième sections (17, 19) sont coniques et la conicité de la deuxième section (17) diffère de la conicité de la troisième section (19).

2. Dispositif de guidage (10) selon la revendication 1, ladite deuxième section (17) étant une section conique convexe et/ou ladite troisième section (19) étant une section conique concave.

3. Dispositif de guidage (10) selon l'une quelconque des revendications précédentes, ladite extrémité proximale de la première section (15) coïncidant avec l'extrémité proximale (12a) du composant effilé (12) et, éventuellement, le diamètre de la première section (15) représentant le diamètre maximal du composant effilé (12).

4. Dispositif de guidage (10) selon l'une quelconque des revendications précédentes, ladite extrémité distale de la troisième section (19) coïncidant avec l'extrémité distale (12b) du composant effilé (12) et éventuellement le diamètre minimal du composant effilé (12) coïncidant avec l'extrémité distale de la troisième section (19).

5. Dispositif de guidage (10) selon l'une quelconque des revendications précédentes, ledit déplacement du composant effilé (12) le long du fil-guide étant contrôlable de manière proximale depuis au-delà de l'extrémité proximale du cathéter vasculaire (22) par le déplacement du moyen de poussée (14).

6. Dispositif de guidage (10) selon la revendication 5, comprenant un raccord (18), pouvant être relié au moyen de poussée (14), permettant de relier de manière amovible le dispositif de guidage (10) à l'extrémité proximale du cathéter vasculaire (22), et éventuellement ledit raccord (18) étant mobile le long du moyen de poussée (14).

7. Dispositif de guidage (10) selon la revendication 6, ledit raccord (18) comprenant un mécanisme de roue libre conçu pour permettre le raccordement du raccord (18) à l'extrémité proximale du cathéter vasculaire (22) sans provoquer de rotation du moyen de poussée (14), et éventuellement ledit raccord (18) comprenant un corps (30) pouvant être relié au moyen de poussée (14) et un anneau de roue libre (24) pour le raccordement au cathéter (22).

8. Dispositif de guidage (10) selon l'une quelconque des revendications 5 à 7, ledit moyen de poussée (14) présentant une section transversale non circulaire.

9. Dispositif de guidage (10) selon la revendication 8, ladite section transversale du moyen de poussée (14) possédant une pluralité de côtés et, éventuellement, ladite section transversale (40) du moyen de poussée (14) possédant quatre côtés.

10. Dispositif de guidage (10) selon la revendication 9, les côtés adjacents étant de longueurs différentes et/ou les coins entre les côtés adjacents étant arrondis.

11. Dispositif de guidage (10) selon l'une quelconque des revendications 9 à 10, ladite pluralité de côtés comprenant un premier côté incurvé (442 ; 542 ; 742 ; 842 ; 942), et éventuellement ledit premier côté incurvé (442 ; 542 ; 742 ; 842 ; 942) s'adaptant à une partie de la circonférence de la surface externe de l'extrémité proximale (12a) du composant effilé (12).

12. Dispositif de guidage (10) selon l'une quelconque des revendications précédentes, comprenant un alésage interne (13a) accessible à travers l'ouverture (13).

13. Dispositif de guidage (10) selon la revendication 12, lorsqu'elle dépend de la revendication 9, ladite pluralité de côtés comprenant un second côté incurvé (441 ; 741 ; 1041) qui s'adapte à une partie de la circonférence de l'alésage interne (13a) du composant effilé (12).

14. Dispositif de guidage (10) selon l'une quelconque des revendications précédentes, ledit moyen de poussée (14) étant agencés de manière à permettre à une partie du fil-guide qui est radialement adjacente au moyen de poussée (14) de rester visible par un opérateur lors de l'utilisation.

15. Kit comprenant :
un dispositif de guidage (10) selon l'une quelconque des revendications précédentes ; et
au moins un élément parmi :
(i) un cathéter vasculaire (22) dans lequel le composant effilé (12) du dispositif de guidage (10) est conçu pour traverser un alésage du cathéter vasculaire (22) et s'étendre à partir d'une extrémité distale (22a) de celui-ci ; et
(ii) un fil-guide dans lequel l'ouverture (13) du composant effilé (12) peut permettre le passage du fil-guide à travers elle de sorte que le composant effilé (12) puisse se déplacer le long du fil-guide.
